Europäisches Patentamt

**(19)** European Patent Office

Office européen des brevets

**(11)** Publication number : **0 387 945 B1**

# **(12)** EUROPEAN PATENT SPECIFICATION

**(45)** Date of publication of patent specification :
03.11.93 Bulletin 93/44

**(51)** Int. Cl.⁵ : **A61K 37/62**

**(21)** Application number : **90200532.1**

**(22)** Date of filing : **06.03.90**

**(54)** **A composition for the treatment of exocrine insufficiency of the pancreas, and the use of said composition.**

**(30)** Priority : **08.03.89 EP 89200589**

**(43)** Date of publication of application :
**19.09.90 Bulletin 90/38**

**(45)** Publication of the grant of the patent :
**03.11.93 Bulletin 93/44**

**(84)** Designated Contracting States :
**BE CH DE DK ES FR GB LI LU NL**

**(56)** References cited :
BE-B- 662 862
DE-A- 3 642 853

**(73)** Proprietor : **Scharpé, Simon Lodewijk**
**Kerkhofstraat 7**
**B-9380 Wieze (BE)**

**(72)** Inventor : **Iliano, Luc Oscar Julien**
**Doolaegepark 20**
**B-9210 Heusden (BE)**
Inventor : **Scharpé, Simon Lodewijk**
**Kerkhofstraat 7**
**B-9380 Wieze (BE)**

**(74)** Representative : **Prins, Hendrik Willem et al**
**Octrooibureau Arnold & Siedsma**
**Sweelinckplein, 1**
**NL-2517 GK The Hague (NL)**

## Description

The present invention relates to a composition for the treatment of exocrine insufficiency of the pancreas, to the use of said composition as a therapeutic agent, and to a method for treating patients suffering from exocrine insufficiency of the pancreas.

More particularly, the invention relates to a composition for alleviating the symptoms of exocrine insufficiency of the pancreas, such as acute of chronic pancreatitis, maldigestion after stomach resection, pancreatectomy and cystic fybrosis.

Treatment of exocrine insufficiency of the pancreas ought to be as easy as treatment of endocrine insufficiency of the thyroid, by administring an extract of the mammalian pancreas, such as porcine pancreas. (See French patent 1,605,287).

Unfortunately adequate treatment is not easy. For instance, in one investigation (Ref. Goodchild M.C., British Medical Journal 1974, iii, page 712) during treatment using porcine pancreatic extract, the faecal excretion of fat ranged from 10% upto 100% in comparison to the intake. Several workers (Ref. Graham D.Y., The New England Journal of Medicine 296, page 1314, 1977) have reported large variations in enzymatic activities of commercial extracts, varying from near zero to satisfactory values.

Even when potent porcine pancreatic extracts (pancreatin) are used for the treatment of pancreatic insufficiency, a correction of the maldigestion and its sequelae may be difficult. Sometimes the pancreatic insufficiency is associated with an insufficient supply of bile-salts (for instance in cancer of the pancreas with obstruction of the bile duct, or after pancreatectomy or gastrectomy).

Mammalian pancreatic enzymes are rapidly and irreversibly denatured by a combination of gastric acid and pepsin. Accordingly administration of a pancreatic extract in powderous form, or in compositions which release the enzymes in a environment in which gastric acid and pepsin are present, will result in an untimely partial or complete denaturation of the enzymes. Unfortunetaly, many patients suffering from pancreatic insuffiency excrete normal, or even increased amounts of gastric juice. Therefore the duodenal contents may be abnormally acid.

Attempts have been made to prevent the undesired destruction of the enzymes of the pancreatic extracts during gastric transit.

DE-A-36 24 853 discloses a drug for therapy of pancreas insufficiency comprising animal pancreatin and/or an enzyme concentrate from Rhizopus arrhizus and/or Aspergillus incorporated in a gastric acid resistant capsule. In the only example porcine pancreatin mixed with sodium hydrogen carbonate and potassium hydrogen carbonate are included in a gastric acid resistant capsule. Twenty capsules a day are used during the daily meals.

This type of composition is not always satisfactory because of several reasons. In patients with cystic fibrosis often a relative hyper-chlorhydria is present and as a consequence the duodenal content may be abnormally acid. This means that the desintegration of the coating in the intestinal tract occurs too late and the enzymatic contents are released in the colon. In patients with hypochlorhydria or when adjuvant anti-acid therapy is applied, the pH of the stomach can rise initially above 5.0. As a consequence desintegration of the coating releases enzymes in the stomach. If pH further, e.g. postprandially, decreases to less than 4.0, pancreatin lipase will be inactivated before being able to function in the duodenum.

In a second attempt the efficacy of the treatment with the pancreatic extract was improved by concomitant administration of omeprazole an antacid inhibiting the gastric acid secretion (Lamers, A.B.H.W. et al., in British Medical Journal 293, page 994, 1986), or by concomitant administration of cimetidine, an $H_2$-receptor antagonist (Regan P.T., New England Journal of Medicine, 297, page 854, 1977).

However, the reduced gastric activity eliminates a natural physical protective barrier and may cause in continuous use bacterial overgrowth (Stockbruegger R.W. et al. Scandinavian Journal of Gastroenterology (Suppl), 20, page 7, 1985). It can also provoke gastroenteral infections (Howden C.w. et al. Gut, 28, page 96, 1987). This induces a supplementary risk especially in cystic fibrosis patents, who are because of the nature of the disease, very susceptible to intestinal infections.

In a third attempt (W. Berndt, Therapiewoche 29, 7095-7102 (1979)) patients suffering from exocrine pancreatic insufficiency were treated with an enzyme substitution composition comprising lipase from Rhizopus proteases and amylase both originating from Aspergillus Oryzae. The fungal lipase is acid-stable, so that in principle any denaturation influenced by gastric acid is overcome.

However, human pancreatic juice contains a properly balanced mixture of enzymes, such as trypsin, chymotrypsin, carboxypeptidases, amylase, cholesterol esterase, ribonuclease and desoxyribonuclease and a wide variety of proteins may be digested. The fungal enzymes used have a rather incomplete spectrum of enzymatic activity so that in comparison with the normal situation various proteins are not or only partially digested. Furthermore the activity of proteases in pancreatic extracts is controlled within rather strict limits by auto-proteolysis, by endogenic inhibitors in order to prevent damage of the intestinal wall.

On the other hand, the presence of cholesterolesterase is also important. This enzyme utilizes a wide variety of substrates ranging from cholesteryl esters to the esters of the fat soluble vitamins A, E, $D_3$, which occur predominantly in natural sources in esterified form and are frequently deficient in patients affected by cystic fibrosis and chronic pancreatitis.

Aspergillus oryzae possess (Sanger F., et al., The Biochemical Journal, 59, page 509, 1955), like many other pathogenic micro-organisms an extracellular post-proline protease activity. This activity is able to cleave the hinge region of IgA, the class of immunoglobulin which predominates in human intestinal tractus (Mulks et al., Infection and Immunity, 55, page 931, 1987) and to inactivate human alpha-1-antitrypsin, a major endogenic protease inhibitor (Morihara K. et al., Journal of Biochemistry, 95, page 795, 1984).

When non-mammalian proteases are used, such as bromelaine, ficine and papaine, their effects are not restricted to the digestion of food proteins. For instance bromelaine may actually increase staetorrhoea because the mucosa of the small intestin may be damaged by bromelaine (Ref. The Lancet No. 8028, page 74, 1977).

Finally, the two metalloproteases, carboxypeptidase A and B active in pancreas extract are, in contrast to the metalloproteases present in Aspergillus oryzae (Morihara K. et al., Archives of Biochemistry and Biophysics, 123, page 572, 1968), characterized by a restricted substrate specificity preventing possible noxious effects.

In conclusion, as stated in the article entitled "Controversies in the Treatment of Exocrine Pancreatic Insufficience" of E.P. DiMagno in Digestive Diseases and Sciences, Vol. 27, page 481-484 (1982) there is still a great need for improved compositions which minimize or eliminate the draw-backs of the afore-mentioned prior art compositions for the treatment of exocrine pancreatic insufficiency.

The object of the invention is a composition for the treatment of exocrine insufficiency of the pancreas, in which a microbial lipase is used in order to overcome the draw-back with respect to acid-instability of human lipase, and in which a mammalian pancreatic extract is used, but only in a much smaller amount in comparison with the prior art compositions, because this pancreatic extract is only used to supplement all pancreatic enzymes but lipase. The enzyme composition of porcine pancreas is almost similar to human pancreas (Desnuelle P., Advances in Enzymology 23, page 129, 1961). Up to now huge amounts of pancreatic extract were used in order to supplement also the normal amount of lipase and to correct for the fraction of the lipase, which is denaturated. The denaturated fraction cannot be estimated in advance. Accordingly, the composition according to the invention for the treatment of exocrine insufficiency of the pancreas comprises as active components a microbial lipase and a mammalian pancreatic extract.

The combination of microbial lipase and mammalian pancreatic extract results in a supplementation of amylolytic and proteolytic enzymes. Accordingly, the glucidolytic intestinal flora, for instance lactobacteriaceae, is maintained. These bacteria exhibit antagonistic effects on pathogenic bacteria (Ref. Pharmaceutical Enzymes, Ed. Ruyssen R., Lauwers A., Story-Scientia, Gent, 1978, page 70). Moreover, the supplementation of amylolytic activity prevents an extended stay of large amounts of osmotically active sugars in the intestinal lumen and the herewith associated defective absorption of water and ions. If an insufficient amount of amylolytic and proteolytic enzymes is supplemented, digestion in the duodenum will lead to an acidification and fermentation of the intestinal contents. Furthermore, the combination in mammalian pancreatic extract between respectively trypsin and carboxy-peptidase B, and chymotrypsin and carboxypeptidase A, ensures an adequate nutritional supply of essential amino acids (Riordan J.F., in Food related Enzymes, ed. J. Whitaker, American Chemical Society 1974, page 223).

Preferably the microbial lipase used is upgraded to a specific activity of more than 125,000, preferably 600,000, more preferably 1,500,000 FIP U/g. Accordingly, by-products are further removed whereas the number of dosing forms is further reduced. The lipase could be obtained from fungi and yeast, such as Aspergillus, Candida, Geotrichum, Mucor, Penicillium, Rhizopus and Sclerotina, and from bacteria, such as Bacillus, Chromobacterium, Pseudomonas and Staphylococcus.

Obviously, these two active components may be administered in one single dosing form comprising an intimate mixture of both components or a mixture of sub-granules, or may be administered as separate dosing forms at the same time or after one another.

The composition according to the invention is suitable for the treatment of acute or chronic pancreatis, of pancreatic or duodenal neoplasms, of maldigestion after stomach or pancreas resection, of cystic fibrosis.

Purification of lipase of Rhizopus

R.a.-lipase was obtained from Rhizopus arrhizus as described in the FR-A-1,605,287. This lyophilized R.a.-lipase powder (40,000-50,000 FIP U/g) was dissolved in water at 2°C. This solution was treated by anion exchange chromatography on Q-Sepharose Fast Flow (trade mark of Pharmacia). A column of a height of 200 mm and a diameter of 50 mm was used. The column was eluted using a gradient of 0-1 M 1,3-[tris(hydroxymethyl)methylaminol]-propane/HCL (pH 7.3). The lipase-active fractions were collected (more that 5,000 FIP U/ml).

The pooled fractions were treated by gelfiltration on Sephacryl- S 100 high Resolution (trade mark of Pharmacia). 0.02 M NaCl + 0,005 M Calcium acetic acid was used as buffer (column height 1,000 mm, internal diameter 50 mm). Fractions containing lipase activity (more than 5,000 FIP U/ml) were pooled.

These pooled fractions were treated by cation exchange chromatography on S Sepharose Fast Flow (trade mark of Pharmacia). A column (height 200 mm, internal diameter 50 mm) was loaded with the pooled fraction and eluted using a gradient of 0-1 M $NH_4$-acetate + 0.005 M Calcium acetate (pH 5.5). Fractions containing lipase activity (more than 5,000 FIP U/ml) were collected. The pooled fractions were lyophilized. The powder obtained had a R.a.-lipase activity of more than 500,000 FIP U/g. By trituration of this powder using lactose, the specific activity required was obtained.

The assay used for the determination of the microbial lipase activity is also disclosed in Pharmaceutical Enzymes, page 210-213.

### Dosing forms.

1. Rhizopus lipase in the form of a lyophilized powder, eventually triturated with lactose for use as such or in an "ex tempore" preparation of a syrop.
2. Hard gelatin capsules nr. 0 (0.67 ml) containing a continuation of more than 40,000 FIP units Rhizopus lipase and more than 200 mg pancreatin (British Pharmacopeia ) in the form of pellets, beads or granulate.
3. Hard gelatin capsules nr. 1 (0.48 ml) or smaller containing more than 50,000 FIP units Rhizopus lipase in the form of pellets, beads or granulate, associated with one or more separate hard gelatin capsules nr. 1 containing more than 225 mg pancreatin in the form of pellets, beads or granulate.
4. Hard gelatin capsules nr. 1 or smaller containing more than 50,000 FIP units Rhizopus lipase in the form of pellets, beads or granulate and one or more separate hard gelatin capsules nr. 0 containing more than 300 mg pancreatin in the form of pellets, beads or granulate.
5. Idem as given in 2-4, but without hard capsules.

### In vitro experiments.

We measured by means of an automatic titration procedure with 0.02 N NaOH, as disclosed in "Pharmaceutical Enzymes", the amount of free fatty acids formed from an olive oil emulsion over a fixed time period at 37°C, pH 7.0, in the presence of 0.025% (final concentration) of sodium taurocholate, by the activity of respectively a preparation of hog pancreas extract, Rhizopus lipase and combinations of both.

We found that combinations of lipase from pancreas extract and Rhizopus results in additional activities and that co-lipase neither activates or inhibits Rhizopus lipase, nor is necessary for Rhizopus lipase activity.

### Clinical trials.

### Trial A.

Patients 6 to 16 years of age suffering from cystic fibrosis were treated during two weeks according to three different protocols.

a. a first group received each meal porcine pancreas extract (pancreatine) at a dosis of 6,000 FIP U lipase, 7,000 FIP U amylase and 500 FIP U protease/galenic dosing form (capsule). The total dosis administered each meal was one galenic dosing form or multiples thereof.

b. a second group received each meal Rhizopus lipase at a dosis of 7,000, 14,000 or 21,000 FIP lipase units per galenic dosing form (capsule), depending on the severity of the insufficiency of the pancreas, the severity of the symptoms, etc. The total dosis administered each meal was one galenic dosing form or multiples thereof.

c. a third group received each meal a combination of porcine pancreas extract and Rhizopus lipase using the same dosis as given in group a and b.

A very clear supplementary therapeutic effect was noticed in the patients belonging to group c in comparison with groups a and b. Patients belonging to group b had some problems due to meteorism and other sequellae of a disturbed intestinal bacterial flora (yeasts), caused by insufficient proteolytic and amyolytic digestion.

Trial B.

a. Type of patients evaluated

10 patients, 5 adults and 5 children, 5 females and 5 males, with established chronic pancreatic insufficiency (cystic fibrosis) and under chronic pancreatin substitution therapy.

b. Duration of the study: 30 days.

c. Dose regimen during the study

A combination, or multiples thereof, (1), a dosis of 50,000 U Rhizopus lipase and (2) porcine pancreas extract (pancreatine) at a dosis per galenic form (capsule) of 6,000 FIP U lipase, 7,000 FIP U amylase and 500 FIP U protease, was given during each meal.

d. Fecal fat excretion

Stool collections were performed during three consecutive days respectively, before the start of the study and at the end of the trial. The daily fecal fat excretion was determined titrimetrically (Van de Kamer et al., Journal of Biological Chemistry, 177, p. 347, 1949).

| patient nr. | sex | age | mean decrease of fecal fat excretion |
|---|---|---|---|
| 1 | F | 29 | 85% |
| 2 | F | 29 | 83% |
| 3 | M | 25 | 69% |
| 4 | F | 24 | 73% |
| 5 | M | 23 | 87% |
| 6 | F | 9 | 81% |
| 7 | M | 9 | 78% |
| 8 | M | 7 | 86% |
| 9 | M | 6 | 72% |
| 10 | F | 5 | 79% |

e. All patients finished the trial and showed a relief of the symptoms of incomplete exocrine pancreatic function. A significant gain in body weight was noticed at the end of the study; in adults minimum 1 kg. In contrast with their condition before the start of the study, the patients were able to enjoy an normal diet without any dietary fat restriction during the period of the trial.

**Claims**

1. Composition for the treatment of exocrine insufficiency of the pancreas, comprising as active components a microbial lipase and a mammalian pancreatic extract, and a pharmaceutically acceptable carrier or diluent.

2. Composition as claimed in claim 1, wherein said microbial lipase is a fungal lipase.

3. Composition as claimed in claim 2, wherein said fungal lipase is Rhizopus lipase.

4. Composition as claimed in claim 1-3, wherein said microbial lipase is upgraded to a specific activity of more than 125,000, preferably 600,000, more preferably 1,500,000 FIP U/g.

5. Composition as claimed in claim 1-4, wherein said mammalian pancreatic extract is a porcine pancreatic extract.

6. Composition as claimed in claim 1-5, comprising more than 40,000 FIP units lipase and at least about 200 mg porcine pancreatic extract.

7. Composition as claimed in claim 6, comprising more than 60,000 FIP units lipase.

8. Composition as claimed in claim 1-7, wherein said active components are contained in a single dosing form.

9. Composition as claimed in claim 1-8, wherein said active components are contained in separate dosing forms associated in a composition packaging.

10. The use of a composition as claimed in claim 1-9, for the manufacture of a medicament for the treatment of exocrine insufficiency of the pancreas.

11. The use as claimed in claim 10, for the treatment of acute or chronic pancreatitis, of pancreatic of duodenal neoplasms, of maldigestion after stomach or pancreas resection, or of cystic fybrosis.

## Patentansprüche

1. Zusammensetzung zur Behandlung von exokriner Pankreasinsuffizienz, als aktive Komponenten eine mikrobische Lipase und einen Säugetier-Pankreasextrakt sowie einen pharmazeutisch annehmbaren Träger oder ein pharmazeutisch annehmbares Verdünnungsmittel umfassend.

2. Zusammensetzung wie in Anspruch 1 beansprucht, worin die mikrobische Lipase eine Pilzlipase ist.

3. Zusammensetzung wie in Anspruch 2 beansprucht, worin die Pilzlipase Rhizopus-Lipase ist.

4. Zusammensetzung wie in den Ansprüchen 1-3 beansprucht, worin die mikrobische Lipase auf eine spezifische Aktivität von mehr als 125000, vorzugsweise 600000, mehr bevorzugt 1500000, FIP-Einheiten/g eingestellt ist.

5. Zusammensetzung wie in den Ansprüchen 1-4 beansprucht, worin der Säugetier-Pankreasextrakt ein Schweinepankreasextrakt ist.

6. Zusammensetzung wie in den Ansprüchen 1-5 beansprucht, umfassend mehr als 40000 FIP-Einheiten Lipase und mindestens ungefähr 200 mg Schweinepankreasextrakt.

7. Zusammensetzung wie in Anspruch 6 beansprucht, umfassend mehr als 60000 FIP-Einheiten Lipase.

8. Zusammensetzung wie in den Ansprüchen 1-7 beansprucht, worin die aktiven Komponenten in einer einzigen Dosierungsform enthalten sind.

9. Zusammensetzung wie in den Ansprüchen 1-8 beansprucht, worin die aktiven Komponenten in getrennten Dosierungsformen, die in einer zusammengesetzten Packung vereinigt sind, enthalten sind.

10. Verwendung einer Zusammensetzung wie in den Ansprüchen 1-9 beansprucht, zur Herstellung eines Medikaments zur Behandlung von exokriner Pankreasinsuffizienz.

11. Verwendung wie in Anspruch 10 beansprucht, für die Behandlung von akuter oder chronischer Pankreatitis, von Pankreas- oder Duodenumneoplasmen, von Verdauungsstörungen nach Magen- oder Pankreasresektion oder von zystischer Fibrose.

## Revendications

1. Composition pour le traitement de l'insuffisance exocrine du pancréas, comprenant une lipase microbienne et un extrait pancréatique de mammifère comme principes actifs et un support ou diluant pharmaceutiquement acceptable.

2. Composition selon la revendication 1, caractérisée en ce que ladite lipase microbienne est une lipase fongique.

3. Composition selon la revendication 2, caractérisée en ce que ladite lipase fongique est une lipase de <u>Rhizopus</u>.

4. Composition selon les revendications 1 à 3, caractérisée en ce que ladite lipase microbienne est concentrée à une activité spécifique supérieure à 125 000 FIP U/g, de préférence supérieure à 600 000 ou encore mieux, supérieure à 1 500 000 FIP U/g.

5. Composition selon les revendications 1 à 4, caractérisée en ce que l'extrait pancréatique de mammifère est un extrait pancréatique de porc.

6. Composition selon les revendications 1 à 5, comprenant plus de 40 000 FIP unités de lipase et au moins environ 200 mg d'extrait pancréatique de porc.

7. Composition selon la revendication 6, comprenant plus de 60 000 FIP unités de lipase.

8. Composition selon les revendications 1 à 7, caractérisée en ce que les principes actifs se présentent sous forme de dose unique.

9. Composition selon les revendications 1 à 8, caractérisée en ce que les principes actifs se présentent sous forme de doses indépendantes rassemblées dans un conditionnement de la composition.

10. Utilisation de la composition selon les revendications 1 à 9 pour préparer un médicament destiné au traitement de l'insuffisance exocrine du pancréas.

11. Utilisation selon la revendication 10, pour traiter les pancréatites chroniques ou aigües, les néoplasmes duodénaux du pancréas, les mauvaises digestions liées à la résection de l'estomac ou du pancréas ou les fibroses kystiques.